# FASCICULE DE BREVET EUROPEEN

(11) **EP 2 753 322 B1**
(45) Date de publication et mention de la délivrance du brevet: **03.08.2016**
(21) Numéro de dépôt: 12769146.7
(22) Date de dépôt: 07.09.2012
(51) Int. Cl.: A61K 31/138, A61K 31/343, A61K 31/704, A61K 33/24, A61P 35/00

(54) **SCHEMA D'ADMINISTRATION DU N-HYDROXY-4-{2-[3-(N,N-DIMETHYLAMINOMETHYL)BENZOFURAN-2-YLCARBONYLAMINO]ETHOXY}BENZAMIDE**
DOSIERSCHEMA VON N-HYDROXY-4-{2-[3-(N,N-DIMETHYLAMINOMETHYL)BENZOFURAN-2-YLCARBONYLAMINO]ETHOXY}BENZAMIDE
DOSAGE REGIMEN OF N-HYDROXY-4-{2-[3-(N,N-DIMETHYLAMINOMETHYL)BENZOFURAN-2-YLCARBONYLAMINO]ETHOXY}BENZAMIDE

(30) Priorité: 08.09.2011 FR 1102727; 08.09.2011 US 201161573585 P
(43) Date de publication de la demande: 16.07.2014
(73) Titulaire: Pharmacyclics, Inc., Sunnyvale, CA 94085 (US)
(72) Inventeur: KLOOS, Ioana, F-92500 Rueil Malmaison (FR); ROBERT, Renata, F-92150 Suresnes (FR); JACQUET-BESCOND, Anne, F-92350 Le Plessis Robinson (FR); DEPIL, Stéphane, F-92130 Issy les Moulineaux (FR); CHENEL, Marylore, F-75014 Paris (FR); FOULIARD, Sylvain, F-75017 Paris (FR); BALASUBRAMANIAN, Sriram, San Carlos, California 94070 (US)
(74) Mandataire: Chajmowicz, Marion
(86) Numéro de dépôt international: PCT/FR2012/052004
(87) Numéro de publication internationale: WO 2013/034863

(56) Documents cités:
- J. J. BUGGY: "CRA-024781: a novel synthetic inhibitor of histone deacetylase enzymes with antitumor activity in vitro and in vivo", MOLECULAR CANCER THERAPEUTICS, vol. 5, no. 5, 1 mai 2006 (2006-05-01), pages 1309-1317, XP055045566, ISSN: 1535-7163, DOI: 10.1158/1535-7163.MCT-05-0442 cité dans la demande
- Anonymous: "PCI-24781 - a novel and potent HDAC inhibitor Non-confidential Overview", , 1 octobre 2009 (2009-10-01), pages 1-3, XP055045978, Extrait de l'Internet: URL:http://www.pharmacyclics.com/pdf/PCI-2 4781_HDAC_Inhibitor_Exec_Overview_Oct09.pd f [extrait le 2012-11-29]
- G. LOPEZ ET AL: "Combining PCI-24781, a Novel Histone Deacetylase Inhibitor, with Chemotherapy for the Treatment of Soft Tissue Sarcoma", CLINICAL CANCER RESEARCH, vol. 15, no. 10, 15 mai 2009 (2009-05-15), pages 3472-3483, XP055045984, ISSN: 1078-0432, DOI: 10.1158/1078-0432.CCR-08-2714
- THOMAS, SCOTT (CORRESPONDENCE) ET AL: "The HDAC inhibitor PCI-24781 potentiates the anti-tumor activity of tamoxifen in ER-positive breast cancer cells.", CANCER RESEARCH, (15 APR 2011) VOL. 71, NO. 8, SUPP. SUPPL. 1. ABSTRACT NUMBER: 2631. MEETING INFO: 102ND ANNUAL MEETING OF THE AMERICAN ASSOCIATION FOR CANCER RESEARCH, AACR 2011. ORLANDO, FL, UNITED STATES. 02 APR 2011-06 APR 2011 ISSN: 0008-5472, ABST2631, 15 avril 2011 (2011-04-15), XP002688224,
- EVENS ANDREW M ET AL: "Phase I Analysis of the Safety and Pharmacodynamics of the Novel Broad Spectrum Historic Deacetylase Inhibitor (HDACi) PCI-24781 in Relapsed and Refractory Lymphoma.", BLOOD, vol. 114, no. 22, novembre 2009 (2009-11), page 1067, XP002688225, & 51ST ANNUAL MEETING OF THE AMERICAN-SOCIETY-OF-HEMATOLOGY; NEW ORLEANS, LA, USA; DECEMBER 05 -08, 2009 ISSN: 0006-4971
- U.S. National Institutes of Health: "PCYC-0401: Study of the Tolerability, Safety, and Pharmacokinetics of CRA-024781 in Cancer Patients", Clinal Trials.gov , 24 août 2010 (2010-08-24), XP002688226, Extrait de l'Internet: URL:http://www.clinicaltrials.gov/ct2/show /NCT00473577?term=PCYC-0401&rank=1 [extrait le 2012-11-29]
- "HDAC Inhibitor Phase I Trial in Advanced Cancer - PCYC-0402", Pharmacyclics , 2010, XP002688227, Extrait de l'Internet: URL:http://www.pharmacyclics.com/clinical_ trial_hdaci_pcyc0402.html [extrait le 2012-11-26]
- U.S. National Institutes of Health: "PCYC-0402: Study of the Safety and Tolerability of Oral Capsule Form of PCI-24781 in Advanced Cancer Patients", ClinicalTrails. gov , 26 septembre 2012 (2012-09-26), XP002688228, Extrait de l'Internet: URL:http://www.clinicaltrials.gov/ct2/show /NCT00562224?term=PCYC-0402&rank=1 [extrait le 2012-11-26]
- FOULIARD SYLVAIN ET AL: "Pharmacokinetic/pharmacodynamic modelling-based optimisation of administration schedule for the histone deacetylase inhibitor abexinostat (S78454/PCI-24781) in phase I", EUROPEAN JOURNAL OF CANCER, vol. 49, no. 13, September 2013 (2013-09), pages 2791-2797, ISSN: 0959-8049(print)

## Description

La présente invention concerne un nouveau schéma d'administration du *N*-hydroxy-4-{2-[3-(*N,N-*diméthylaminométhyl)benzofuran-2-ylcarbonylamino]éthoxy}benzamide de formule (I) : 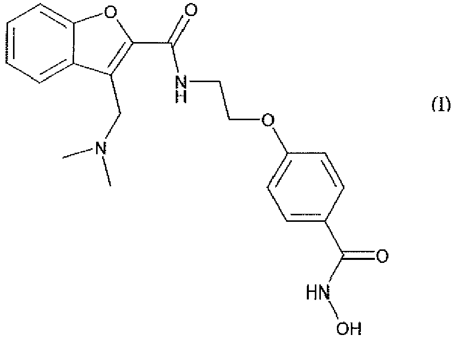 ou d'un de ses sels d'addition à un acide ou à une base pharmaceutiquement acceptable, seul ou en association avec un traitement de chirurgie, de chimiothérapie, d'hormonothérapie ou avec la radiothérapie, pour le traitement du cancer, tel que défini dans les revendications.

Le *N*-hydroxy-4-{2-[3-(*N,N*-diméthylaminométhyl)benzofuran-2-ylcarbonylamino] éthoxy}benzamide est un inhibiteur puissant des histones-déacétylases (HDAC) décrit dans la demande de brevet WO2004/092115. Il permet d'inhiber la croissance cellulaire et induit l'apoptose dans des cellules tumorales cultivées *in vitro*, et inhibe la croissance tumorale *in vivo* dans des modèles de xénogreffes (Buggy et al Mol. Cancer Ther 2006 5(5) 1309). Son profil pharmacologique lui confère un intérêt thérapeutique majeur dans le traitement du cancer.

Dans la présente invention, il a été établi que le *N-*hydroxy-4-{2-[3-(*N,N-*diméthylaminométhyl)benzofuran-2-ylcarbonylamino]éthoxy}benzamide de formule (I) présentait un index thérapeutique très avantageux dans le traitement du cancer lorsqu'il était administré pendant 4 jours consécutifs, cette période étant suivie de 3 jours consécutifs sans aucune administration du composé de formule (I).

Plus précisément, il a été montré que la dose maximale tolérée du *N*-hydroxy-4-{2-[3-(*N,N-*diméthylaminométhyl)benzofuran-2-ylcarbonylamino]éthoxy}benzamide augmentait considérablement chez les patients atteints d'un cancer lorsqu'il était administré pendant 4 jours consécutifs, cette période étant suivie de 3 jours consécutifs sans aucune administration du composé de formule (I). Ce cycle d'administration est répété autant de fois que nécessaire pour le traitement du cancer. Alternativement, ces cycles hebdomadaires d'administration peuvent être périodiquement entrecoupés par une semaine sans aucun traitement.

Ce schéma d'administration permet d'envisager une meilleure utilisation du *N*-hydroxy-4-{2-[3-(*N,N*-diméthylaminométhyl)benzofuran-2-ylcarbonylamino]éthoxy}benzamide dans la mesure où il permet de minimiser la toxicité plaquettaire inhérente au produit tout en maintenant une exposition suffisante pour le traitement du cancer.

La présente invention concerne le *N*-hydroxy-4-{2-[3-(*N,N*-diméthylaminométhyl) benzofuran-2-ylcarbonylamino]éthoxy}benzamide de formule (I) : 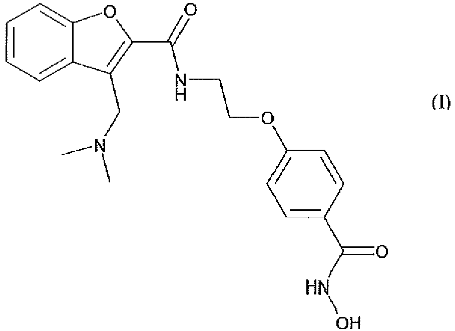 ou un de ses sels d'addition à un acide ou à une base pharmaceutiquement acceptable, seul ou en association avec un traitement de chirurgie, de chimiothérapie, d'hormonothérapie ou avec la radiothérapie, pour le traitement du cancer chez le patient humain, caractérisé en ce qu'il est administré pendant 4 jours consécutifs, cette période étant suivie de 3 jours consécutifs sans aucune administration du composé de formule (I), étant entendu que le traitement de chimiothérapie n'est pas le FOLFOX (oxaliplatine/acide folinique/5-fluorouracil).

Préférentiellement, le *N*-hydroxy-4-{2-[3-(*N,N*-diméthylaminométhyl)benzofuran-2-ylcarbonylamino]éthoxy}benzamide est utilisé sous la forme d'un chlorhydrate dans le cadre de l'invention.

Le *N*-hydroxy-4-{2-[3-(*N,N*-diméthylaminométhyl)benzofuran-2-ylcarbonylaminol] éthoxy}benzamide est avantageusement administré pendant 4 jours consécutifs au rythme de 2 prises quotidiennes, cette période étant suivie de 3 jours consécutifs sans aucune administration du composé de formule (I).

Dans certaines variantes, le cancer traité selon le schéma d'administration de l'invention peut être un carcinome, une tumeur, un néoplasme, un lymphome, un mélanome, un gliome, un sarcome, ou un blastome. Le schéma d'administration selon l'invention est particulièrement utile pour le traitement des tumeurs solides, et plus particulièrement encore pour le traitement du cancer du sein. Le carcinome épithélial de l'ovaire, le carcinome du tube fallopien et le carcinome primaire péritonéal sont aussi spécifiquement visés.

De manière préférentielle, le *N-*hydroxy-4-{2-[3-(*N,N-*diméthylaminométhyl)benzofuran-2-ylcarbonylamino] éthoxy}benzamide est administré selon le régime d'administration de l'invention en association avec un traitement de chimiothérapie, d'hormonothérapie ou avec la radiothérapie.

Alternativement, le *N-*hydroxy-4-{2-[3-(*N,N-*diméthylaminométhyl)benzofuran-2-ylcarbonylamino] éthoxy}benzamide est administré pendant 4 jours consécutifs, cette période étant suivie de 3 jours consécutifs sans aucune autre administration de ce composé, en association avec un traitement de chimiothérapie choisi parmi la doxorubicine, la doxorubicine liposomale pégylée, le cisplatine, le cyclophosphamide, le paclitaxel, le carboplatine, carboplatine/paclitaxel, cisplatine/doxorubicine/cyclophosphamide, et doxorubicine liposomale pégylée/cisplatine. Plus préférentiellement, le traitement de chimiothérapie associé est la doxorubicine, la doxorubicine liposomale pégylée, ou le cisplatine.
De manière alternative, le *N-*hydroxy-4-{2-[3-(*N,N-*diméthylaminométhyl)benzofuran-2-ylcarbonylamino]éthoxy}benzamide est administré pendant 4 jours consécutifs, cette période étant suivie de 3 jours consécutifs sans aucune autre administration de ce composé, en association avec le tamoxifène comme traitement d'hormonothérapie.

Dans un mode de réalisation préféré, le *N-*hydroxy-4-{2-[3-(*N,N-*diméthylaminométhyl)benzofuran-2-ylcarbonylamino]éthoxy}benzamide est administré sous forme orale.

La posologie utile varie selon le sexe, l'âge et le poids du patient, la voie d'administration, la nature du cancer et des traitements éventuellement associés et s'échelonne entre 30 mg/m² et 210 mg/m² de chlorhydrate de *N*-hydroxy-4-{2-[3-(*N,N-*diméthylaminométhyl)benzo-furan-2-ylcarbonylamino]éthoxy}benzamide par jour. Plus généralement, la posologie utile s'échelonne entre 20 mg et 480 mg de *N*-hydroxy-4-{2-[3-(*N,N-*diméthylaminométhyl)benzofuran-2-ylcarbonylamino]éthoxy} benzamide exprimée en équivalent base par jour.

### Etude clinique 1: (N-hydroxy-4-{2-[3-(N,N-diméthylaminométhyl)benzofuran-2-ylcarbonylamino]éthoxy}benzamide en monothérapie)

Une étude clinique a été initiée pour tester la toxicité et l'efficacité du chlorhydrate de *N-*hydroxy-4-{2-[3-(*N,N-*diméthylaminométhyl)benzofuran-2-ylcarbonylamino]éthoxy} benzamide. 36 patients atteints de tumeurs solides ont été inclus et traités dans cette étude. Un premier schéma d'administration a été testé sur un cycle de trois semaines pendant 14 jours consécutifs, à raison de 2 prises *po* espacées de 4 h, suivi d'une semaine sans traitement. Quatre paliers de doses ont été successivement testés de 30 à 75 mg/m² (doses exprimées en sel de chlorhydrate) deux fois par jour.
Les profils d'acceptabilité des différents paliers de dose du *N-*hydroxy-4-{2-[3-(*N,N-*diméthylaminométhyl)benzofuran-2-ylcarbonylamino]éthoxy}benzamide ont été évalués (toxicités hématologiques en particulier) à l'issue du premier cycle.

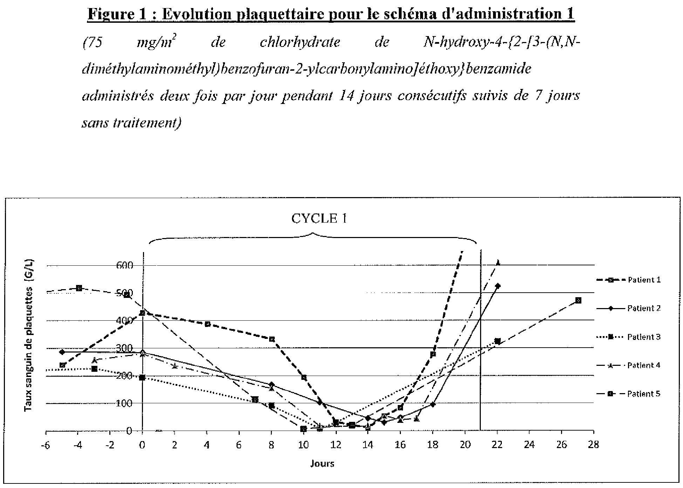

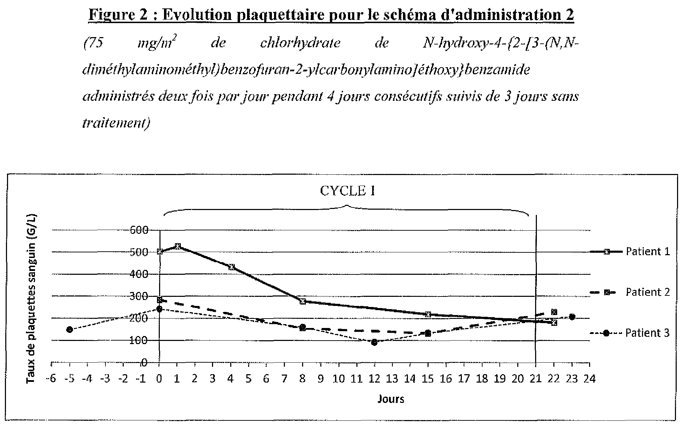

Les résultats ont montré que toutes les toxicités limitant l'augmentation de dose étaient des thrombopénies grade 4 (taux sanguin de plaquettes inférieur à 25 giga par litre), réversibles après l'arrêt du traitement du *N-*hydroxy-4-{2-[3-(*N,N-*diméthylaminométhyl)benzofuran-2-ylcarbonylamino]éthoxy}benzamide (voir la Figure 1). La dose maximale tolérée suivant ce premier schéma d'administration a été établie à 75 mg/m² deux fois par jour.
Pour éviter les diminutions importantes du nombre de plaquettes observées avec ce schéma, le protocole d'étude a été amendé afin de proposer un nouveau schéma d'administration : 4 jours consécutifs, à raison de 2 prises *po* espacées de 4h, puis 3 jours sans traitement, ce schéma étant appliqué pour chacune des 3 semaines correspondant à un cycle d'administration. Ce cycle peut être répété autant de fois que nécessaire pour le traitement du cancer. Ce nouveau schéma a montré moins de toxicité plaquettaire que le schéma précédent et a permis de traiter des patients à des niveaux de doses supérieures (90 à 105 mg/m² deux fois par jour). A titre de comparaison, la Figure 2 montre que le deuxième schéma d'administration est nettement moins délétère que le premier du point de vue du taux sanguin de plaquettes à la dose de 75 mg/m² deux fois par jour. Dans le deuxième schéma d'administration, la dose recommandée et la dose maximale tolérée ont été établies à 90 mg/m² et 105 mg/m² deux fois par jour, respectivement.

### Etude clinique 2: N-hydroxy-4-{2-[3-(N,N-diméthylaminométhyl)benzofuran-2-ylcarbonylamino]éthoxy}benzamide en combinaison avec le cisplatine)

Une étude clinique de phase I pour tester l'association du chlorhydrate de *N*-hydroxy-4-{2-[3-(*N,N*-diméthylaminométhyl)benzofuran-2-ylcarbonylamino]éthoxy}benzamide avec le cisplatine est réalisée sur une quarantaine de patients atteints de diverses tumeurs solides. Les patients reçoivent au maximum six cycles de traitement en association, chacun des cycles se déroulant de la manière suivante : le chlorhydrate de *N-*hydroxy-4-{2-[3*-*(*N,N-*diméthylaminométhyl)benzofuran-2-ylcarbonylamino]éthoxy}benzamide est administré pendant 4 jours consécutifs, suivis de 3 jours consécutifs sans traitement, et ce pendant les deux premières semaines d'un cycle de trois semaines. Le cisplatine est administré le troisième jour de chaque cycle de traitement. Aucun traitement n'est administré pendant la troisième semaine du cycle.

Les jours concernés, la dose journalière de chlorhydrate de *N*-hydroxy-4-{2-[3-(*N,N-*diméthylaminométhyl)benzofuran-2-ylcarbonylamino]éthoxy}benzamide est comprise entre 80 et 320 mg (exprimée en équivalent base), à raison de deux prises *p.o.* espacées de 4 h. Le cisplatine est administré à la dose fixe de 75 mg/m².
Au bout d'un cycle de traitement, la toxicité du protocole associant le chlorhydrate de *N-*hydroxy-4-{2-[3-(*N,N-*diméthylaminométhyl)benzofuran-2-ylcarbonylamino]éthoxy} benzamide et le cisplatine est évaluée. Si aucune toxicité rédhibitoire n'est observée, le patient continue le traitement. Tous les deux cycles, l'efficacité du traitement est appréciée via l'évaluation de la réponse tumorale (CT-scan, IRM,...). Le profil d'acceptabilité est également évalué (toxicités hématologiques et cardiaques en particulier). Le patient continue le traitement en combinaison pendant six cycles au maximum.

### Etude clinique 3: (N-hydroxy-4-{2-[3-(N,N-diméthylaminométhyl)benzofuran-2-ylcarbonylamino]éthoxy}benzamide en combinaison avec la doxorubicine)

Une étude clinique de phase I pour tester l'association du chlorhydrate de *N*-hydroxy-4-{2-[3-(*N,N-*diméthylaminométhyl)benzofuran-2-ylcarbonylamino]éthoxy}benzamide avec la doxorubicine dans le traitement de tumeurs solides avancées est réalisée sur environ quarante patients. Les patients reçoivent au maximum six cycles de traitement en association, chacun des cycles se déroulant de la manière suivante : le chlorhydrate de *N-*hydroxy-4-{2-[3-(*N,N*-diméthylaminométhyl)benzofuran-2-ylcarbonylamino]éthoxyl} benzamide est administré pendant 4 jours consécutifs, suivis de 3 jours consécutifs sans traitement, et ce pendant les trois premières semaines d'un cycle de quatre semaines. La doxorubicine est infusée le troisième jour des trois premières semaines du cycle. Aucun traitement n'est administré pendant la quatrième semaine du cycle.
Les jours concernés, la dose journalière de chlorhydrate de *N*-hydroxy-4-{2-[3-(*N,N-*diméthylaminométhyl)benzofuran-2-ylcarbonylamino]éthoxy}benzamide est comprise entre 60 et 150 mg/m² (doses exprimées en sel de chlorhydrate), à raison de deux prises *p.o.* espacées de 4h. La doxorubicine est administrée à la dose fixe de 25 mg/m².
Au bout d'un cycle de traitement, la toxicité du protocole associant le chlorhydrate de *N-*hydroxy-4-{2-[3-(*N,N-*diméthylaminométhyl)benzofuran-2-ylcarbonylamino]éthoxy} benzamide et la doxorubicine est évaluée. Si aucune toxicité rédhibitoire n'est observée, le patient continue le traitement. Tous les deux cycles, l'efficacité du traitement est appréciée via l'évaluation de la réponse tumorale (CT-scan, IRM,...). Le profil d'acceptabilité est également évalué (toxicités hématologiques et cardiaques en particulier). Le patient continue le traitement en combinaison pendant six cycles au maximum.

### Etude clinique 4: N-hydroxy-4-{2-[3-(N,N-diméthylaminométhyl)benzofuran-2-ylcarbonylamino]éthoxy}benzamide en combinaison avec la doxorubicine liposomale pégylée)

Une étude clinique de phase I pour tester l'association du chlorhydrate de *N*-hydroxy-4-{2-[3-(*N,N*-diméthylaminométhyl)benzofuran-2-ylcarbonylamino]éthoxy}benzamide avec la doxorubicine liposomale pégylée est réalisée sur environ soixante-dix patients dans le traitement du carcinome épithélial de l'ovaire, du carcinome du tube fallopien ou du carcinome primaire péritonéal, ces carcinomes présentant une résistance primaire ou une sensibilité partielle au platine. Les patients sont soumis à des cycles de traitement en association. Deux schémas d'administration sont testés :
- Schéma 1 : le chlorhydrate de *N*-hydroxy-4-{2-[3-(*N,N-*diméthylaminométhyl)benzofuran-2-ylcarbonylamino]éthoxy}benzamide est administré pendant 4 jours consécutifs, suivis de 3 jours consécutifs sans traitement, et ce pendant les trois premières semaines d'un cycle de quatre semaines. La doxorubicine liposomale pégylée est infusée le troisième jour de la première semaine du cycle. Aucun traitement n'est administré pendant la quatrième semaine du cycle.
- Schéma 2 : le chlorhydrate de *N*-hydroxy-4-{2-[3*-*(*N,N-*diméthylaminométhyl)benzofuran-2-ylcarbonylamino]éthoxy}benzamide est administré pendant 4 jours consécutifs, suivis de 3 jours consécutifs sans traitement, et ce pendant la première semaine d'un cycle de quatre semaines. La doxorubicine liposomale pégylée est infusée le troisième jour de la première semaine du cycle. Aucun traitement n'est administré pendant les trois dernières semaines du cycle.
Les jours concernés, la dose journalière de chlorhydrate de *N*-hydroxy-4-{2-[3-(*N,N-*diméthylaminométhyl)benzofuran-2-ylcarbonylamino]éthoxy}benzamide est comprise entre 60 et 150 mg/m² (doses exprimées en sel de chlorhydrate), à raison de deux prises *^{p.o}*. espacées de 4 h. La doxorubicine liposomale pégylée est administrée à la dose fixe de 40 mg/m².
Au bout d'un cycle de traitement, la toxicité du protocole associant le chlorhydrate de *N-*hydroxy-4-{2-[3-(*N,N*-diméthylaminométhyl)benzofuran-2-ylcarbonylamino]éthoxy} benzamide et la doxorubicine liposomale pégylée est évaluée. Si aucune toxicité rédhibitoire n'est observée, le patient continue le traitement. Tous les deux cycles, l'efficacité du traitement est appréciée via l'évaluation de la réponse tumorale (CT-scan, IRM,...). Le profil d'acceptabilité est également évalué (toxicités hématologiques et cardiaques en particulier).

### Etude clinique 5: (N-hydroxy-4-{2-[3-(N,N-diméthylaminométhyl)benzofuran-2-ylcarbonylamino]éthoxy}benzamide en combinaison avec le tamoxifène)

Une étude clinique de phase I pour tester l'association du chlorhydrate de *N*-hydroxy-4-{2-[3-(*N,N*-diméthylaminométhyl)benzofuran-2-ylcarbonylamino]éthoxy}benzamide avec le tamoxifène dans le traitement du cancer du sein avancé est réalisée sur environ 40 patients. Les patients sont soumis à des cycles de traitement en association, chacun des cycles se déroulant de la manière suivante : le chlorhydrate de *N*-hydroxy-4-{2-[3-(*N,N-*diméthylaminométhyl)benzofuran-2-ylcarbonylamino]éthoxy}benzamide est administré pendant 4 jours consécutifs, suivis de 3 jours consécutifs sans traitement. Le tamoxifène est administré quotidiennement pendant toute la durée du traitement.
Les jours concernés, la dose journalière de chlorhydrate de *N*-hydroxy-4-{2-[3-(*N,N-*diméthylaminométhyl)benzofuran-2-ylcarbonylamino]éthoxy}benzamide est comprise entre 160 et 320 mg (exprimée en équivalent base), à raison de deux prises *p.o*. espacées de 4 h. Le tamoxifène est administré quotidiennement à la dose fixe de 20 mg.
Au bout de deux cycles de traitement, la toxicité du protocole associant le chlorhydrate de *N*-hydroxy-4-{2-[3-(*N,N*-diméthylaminométhyl)benzofuran-2-ylcarbonylamino]éthoxy} benzamide et le tamoxifène est évaluée. Si aucune toxicité rédhibitoire n'est observée, le patient continue le traitement. Tous les deux cycles, l'efficacité du traitement est appréciée via l'évaluation de la réponse tumorale (CT-scan, IRM,...). Le profil d'acceptabilité est également évalué (toxicités hématologiques et cardiaques en particulier).

Les diverses études cliniques ci-dessus montrent qu'un schéma d'administration dans lequel le *N-*hydroxy-4-{2-[3-(*N,N-*diméthylaminométhyl)benzofuran-2-ylcarbonylamino] éthoxy}benzamide est administré pendant 4 jours consécutifs, cette période étant suivie de 3 jours consécutifs sans aucune administration, permet de minimiser la toxicité plaquettaire inhérente au produit et d'augmenter ainsi la dose thérapeutique pour le traitement du cancer.

## Revendications

1. *N*-hydroxy-4-{2-[3-(*N,N*-diméthylaminométhyl)benzofuran-2-ylcarbonylamino] éthoxy}benzamide de formule (I) : 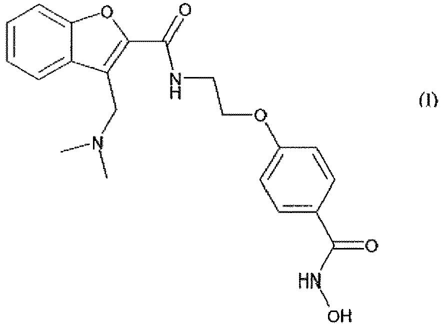 ou un de ses sels d'addition à un acide ou à une base pharmaceutiquement acceptable, seul ou en association avec un traitement de chirurgie, de chimiothérapie, d'hormonothérapie ou avec la radiothérapie, pour une utilisation dans le traitement du cancer chez le patient humain, **caractérisé en ce qu'**il est administré pendant 4 jours consécutifs, cette période étant suivie de 3 jours consécutifs sans aucune administration du composé de formule (I), étant entendu que le traitement de chimiothérapie n'est pas le FOLFOX (oxaliplatine/acide folinique/5-fluorouracil).

2. Composé de formule (I) pour une utilisation selon la revendication 1 **caractérisé en ce qu'**il est utilisé sous la forme d'un chlorhydrate.

3. Composé de formule (I) pour une utilisation selon la revendication 1 ou 2 **caractérisé en ce qu'**il est administré pendant 4 jours consécutifs au rythme de 2 prises quotidiennes, cette période étant suivie de 3 jours consécutifs sans aucune administration du composé de formule (I).

4. Composé de formule (I) pour une utilisation selon l'une des revendications 1 à 3 administré en association avec un traitement de chimiothérapie, d'hormonothérapie ou avec la radiothérapie.

5. Composé de formule (I) pour une utilisation selon l'une des revendications 1 à 4 dans le traitement du carcinome, des tumeurs, du néoplasme, du lymphome, du mélanome, du gliome, du sarcome, ou du blastome.

6. Composé de formule (I) pour une utilisation selon l'une des revendications 1 à 5 dans le traitement des tumeurs solides.

7. Composé de formule (I) pour une utilisation selon l'une des revendications 1 à 5 dans le traitement du cancer du sein.

8. Composé de formule (I) pour une utilisation selon l'une des revendications 1 à 5 dans le traitement du carcinome épithélial de l'ovaire, du carcinome du tube fallopien et du carcinome primaire péritonéal.

9. Composé de formule (I) pour une utilisation selon l'une des revendications 1 à 5 **caractérisé en ce que** le traitement de chimiothérapie est le cisplatine, la doxorubicine ou la doxorubicine liposomale pégylée.

10. Composé de formule (I) pour une utilisation selon l'une des revendications 1 à 5 **caractérisé en ce que** le traitement d'hormonothérapie est le tamoxifène.

## Patentansprüche

1. *N-*hydroxy-4-{2-[3-(*N,N-*dimethylaminomethyl)benzofuran-2-ylcarbonylamino]-ethoxy}-benzamid der Formel I: 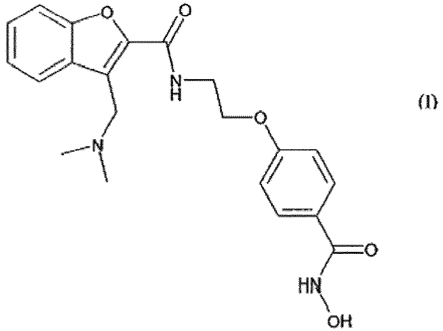 oder eines seiner Additionssalze mit einer Säure oder pharmazeutisch verträglichen Base, allein oder in Kombination mit chirurgischer Behandlung oder Chemotherapie, Hormontherapie oder Radiotherapie, zur Verwendung bei der Behandlung von Krebs im menschlichen Patienten, **dadurch gekennzeichnet, dass** es für 4 aufeinanderfolgende Tage verabreicht wird, wobei auf diesen Zeitraum 3 aufeinanderfolgende Tage ohne Verabreichung der Verbindung der Formel I folgen, wobei es sich versteht, dass die Chemotherapiebehandlung nicht mit FOLFOX (Oxaliplatin/Folinsäure/5-Fluoruracil) stattfinden sollte.

2. Verbindung der Formel I für eine Verwendung nach Anspruch 1, **dadurch gekennzeichnet, dass** sie in der Form eines Hydrochlorids verwendet wird.

3. Verbindung der Formel I für eine Verwendung nach Anspruch 1 oder 2, **dadurch gekennzeichnet, dass** sie für 4 aufeinanderfolgende Tage in einer Rate von 2 Tagesdosen verabreicht wird, wobei auf diesen Zeitraum 3 aufeinanderfolgende Tage ohne Verabreichung der Verbindung der Formel I folgen.

4. Verbindung der Formel I zur Verwendung nach einem der Ansprüche 1 bis 3, die in Kombination mit Chemotherapie, Hormontherapie oder Radiotherapie verabreicht wird.

5. Verbindung der Formel I zur Verwendung nach einem der Ansprüche 1 bis 4 bei der Behandlung von Karzinom, Tumoren, Neoplasie, Lymphom, Melanom, Gliom, Sarkom oder Blastom.

6. Verbindung der Formel I zur Verwendung nach einem der Ansprüche 1 bis 5 bei der Behandlung von soliden Tumoren.

7. Verbindung der Formel I zur Verwendung nach einem der Ansprüche 1 bis 5 bei der Behandlung von Brustkrebs.

8. Verbindung der Formel I zur Verwendung nach einem der Ansprüche 1 bis 5 bei der Behandlung von Epitheleierstockkarzinom, Eileiterkarzinom und primärem Peritonealkarzinom.

9. Verbindung der Formel I zur Verwendung nach einem der Ansprüche 1 bis 5, **dadurch gekennzeichnet, dass** die Chemotherapiebehandlung Cisplatin, Doxorubicin and pegyliertes liposomales Doxorubicin ist.

10. Verbindung der Formel I zur Verwendung nach einem der Ansprüche 1 bis 5, **dadurch gekennzeichnet, dass** die Hormontherapiebehandlung Tamoxifen ist.

## Claims

1. *N*-hydroxy-4-{2-[3-(*N,N*-dimethylaminomethyl) benzofuran-2-ylcarbonylamino] ethoxy} benzamide of formula I: 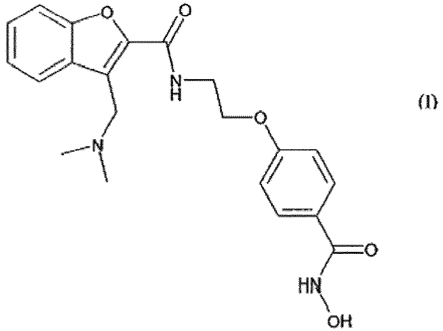 or one of its addition salts with an acid or a pharmaceutically acceptable base, alone or in combination with surgical treatment, or chemotherapy, hormonal therapy, or radiotherapy, for use in treating cancer in the human patient, **characterized in that** it is administered for 4 consecutive days, this period being followed by 3 consecutive days without administration of the compound of formula I, it being understood that the chemotherapy treatment should not be with FOLFOX (oxaliplatin/folinic acid/5-fluorouracil).

2. Formula I compound for a use in accordance with Claim 1 **characterized in that** it is used in the form of a hydrochloride.

3. Formula I compound for a use in accordance with Claim 1 or 2 **characterized in that** it is administered for 4 consecutive days at the rate of 2 daily doses, this period being followed by 3 consecutive days without administration of the Formula I compound.

4. Formula I compound for use in accordance with any of Claims 1 to 3 administered in combination with chemotherapy, hormonal therapy or radiotherapy.

5. Formula I compound for use in accordance with any of Claims 1 to 4 in the treatment of carcinoma, tumors, neoplasm, lymphoma, melanoma, glioma, sarcoma, or blastoma.

6. Formula I compound for use in accordance with any of Claims 1 to 5 in the treatment of solid tumors.

7. Formula I compound for use in accordance with any of Claims 1 to 5 in the treatment of breast cancer.

8. Formula I compound for use in accordance with any of Claims 1 to 5 in the treatment of epithelial ovarian carcinoma, carcinoma of the fallopian tube and primary peritoneal carcinoma.

9. Formula I compound for use in accordance with any of Claims 1 to 5 **characterized in that** the chemotherapy treatment is cisplatin, doxorubicin and pegylated liposomal doxorubicin.

10. Formula I compound for use in accordance with any of Claims 1 to 5 **characterized in that** the hormone therapy treatment is tamoxifen.
